# EUROPÄISCHE PATENTSCHRIFT

(11) **EP 2 441 333 B1**
(45) Veröffentlichungstag und Bekanntmachung des Hinweises auf die Patenterteilung: **04.05.2016**
(21) Anmeldenummer: 11008166.8
(22) Anmeldetag: 10.10.2011
(51) Int. Cl.: A23G 3/48, A23G 4/06, A61K 9/00, A61K 36/185, A61K 36/28, A61K 36/25, A61K 36/09, A61K 36/736, A61K 36/738, A61K 36/734, A61K 36/71, A61K 36/534, A61K 36/315

(54) **KAUFÄHIGE TABLETTE**
CHEWABLE TABLET
COMPRIMÉ À MÂCHER

(30) Priorität: 14.10.2010 DE 102010048283
(43) Veröffentlichungstag der Anmeldung: 18.04.2012
(73) Patentinhaber: SCHAPER & BRÜMMER GMBH & CO. KG, 38259 Salzgitter (DE)
(72) Erfinder: Meyer, Andreas, 38259 Salzgitter (DE); Tegtmeier, Martin, Dr., 38640 Goslar (DE)
(74) Vertreter: Lins, Edgar

(56) Entgegenhaltungen:
- EP-A1- 1 287 828
- EP-A2- 0 169 977
- EP-A2- 1 886 690
- WO-A1-88/09178
- WO-A1-2009/129833
- DE-A1- 2 721 014
- JP-A- 11 246 426
- JP-A- 2005 263 751
- JP-A- 2010 189 334
- NL-C1- 1 003 751
- RU-C1- 2 182 011
- US-A1- 2003 157 206
- US-A1- 2004 132 816
- US-A1- 2004 156 926
- Noweda: "Nahrungsergänzungsmittel-Verzeichnis für Deutschland NEM-2007", 2007, Wissenschaftliche Verlagsgesellschaft mbH, Stuttgart, XP002667338, ISBN: 3-8047-2195-8 * pages 332-335, 337-340, 353-354, 359-361, 370, 379, 385-386, 389. * * Seite 337 - Seite 340 *

## Beschreibung

Die Erfindung betrifft eine Kautablette mit einem kaufähigen, sich im Mund eines Menschen nur nach einem ununterbrochenen Kauen über mehr als eine Minute auflösenden Trägermaterial.

Kautabletten dieser Art dienen ursprünglich dazu, im Mund- und Rachenraum ein Frischegefühl zu erzeugen. Daher sind diese Kautabletten überwiegend mit Pfefferminz- und ähnlichen Aromen versehen.

Seit einiger Zeit sind auch Kautabletten bekannt geworden, die auch als Arzneimittel verwendet werden können. Bekannte Beispiele sind Präparate zur Unterstützung beim Abgewöhnen des Rauchens oder zur Behandlung von Reisekrankheiten in Form von Nikotin- oder dimenhydrinathaltigen Kaugummis. Hierfür sind auch spezielle Formulierungen, wie beispielsweise mehrschichtige Kautabletten, entwickelt worden, wie beispielsweise in US 2006/0039872 A1 beschrieben worden ist. Der Sinn der Applikation von Wirkstoffen über eine Kautablette besteht darin, den Wirkstoff über eine gewisse Applikationsdauer dem Körper zuzuführen. Dabei findet eine Resorption über die Mundschleimhaut und ggf. über den Verdauungstrakt statt, wenn der Wirkstoff mit dem beim Kauen gebildeten Speichel heruntergeschluckt wird und in den Magen gelangt.

WO 2009/0129833 A1 offenbart eine Pflanzenextraktkombination aus Gingko und Ginseng als Grundwirkstoffe, wobei der Gingko-Extrakt mit einem Aceton-Wasser-Gemisch und der Ginseng-Extrakt mit einem Alkohol-Wasser-Gemisch hergestellt wird. Den Extrakten kann ein Crataegus-Pulver in Form von gemahlenen Blättern, Blüten und Früchten oder in Form eines Cratagegus-Extrakts beigemischt werden. Die Wirkstoffkombination kann als Arzneimittel oder Nahrungsergänzungsmittel in Form von Dragees, Tabletten, Filmtabletten, Kapseln usw. verabreicht werden. Die Darreichungsform als Kautablette ist nicht beschrieben.

EP 1 886 690 A2 beschreibt eine insbesondere pharmazeutische Zusammensetzung mit den Bestandteilen Okoubaka, 3, 3', 4', 5, 7-Pentahydroxy-Flavon, Galactosidase und Pyridoxin. Okoubaka wird dabei vorzugsweise aus der Rinde des im westafrikanischen Regenwald beheimateten Baumes Okoubaka aubervillei gewonnen und bevorzugt in homöopatischen Mengen eingesetzt. Die Verabreichung erfolgt vorzugsweise mittels Filmtabletten.

RU 2 182 011 C1 offenbart ein Nahrungsergänzungsmittel in Tablettenform, das einen Trockenextrakt aus Echinacea und physiologisch akzeptablen Füllstoffen, u. a. aus getrocknetem Echinaceagras und Trockenextrakt aus Echinaceagras, enthält.

EP 1 278 828 A1 offenbart eine Mischung zur Teezubereitung zur Behandlung von Verstopfungen und Darmträgheit. Zu Testzwecken ist eine Suspension der Zusammensetzung initiiert worden.

JP 11-246426 A offenbart Pfefferminze als Antioxidationsmittel. Die Herstellung erfolgt durch Trocknung oder Extraktion.

WO 88/09178 A1 offenbart ein Pharmazeutikum, das aus zerkleinerten, pflanzlichen Bestandteilen zusammengepresst sein kann. Die pflanzlichen Bestandteile sind zu einem Pulver zerkleinert. Die Darreichungsform ist eine trockene Tablette oder ein Aufgussbeutel zur Erzeugung eines Tees. Die Tablette soll sich leicht auflösen können.

JP 2010-189334 A offenbart eine Tablette, die ein Acerola-Pulver enthält.

US 2004/0156926 A1 offenbart ein Nahrungsergänzungsmittel, das Astaxanthin, Cimicifuga, Echinacea und Phytosterole enthält. Cimicifuga kann als Pulver der gemahlenen Wurzel oder als Extrakt bereitgestellt werden, während Echinacea nur als Extrakt verwendet wird. Die Verabreichung erfolgt in Form von Pillen, Tabletten oder Kapseln.

US 2004/0132816 A1 beschreibt Getränke, die mit Bioflavoloide enthaltenden Extrakten von verschiedenen Pflanzen der Gattung Crataegus hergestellt sind. DE 27 21 014 A1 offenbart ein Arzneimittel, das rechtsdrehende Milchsäure und Echinacea enthält. Das Echinacea-Pulver wird durch Trocknung durch Teilen der Pflanzen oder gesamten Pflanzen und anschließende Pulverisierung erhalten. Das erhaltene Pulver wird in Kapseln abgefüllt. Die Verabreichung kann ferner in Form von Tabletten, Lutschbonbons, Kapseln, Elixieren, Suspensionen, Tinkturen oder Salben oder in Form von Pudern erfolgen.

US 2003/0157206 A1 offenbart die Herstellung eines Mundpflegemittels aus Cimicifuga-Wurzeln. Die Cimicifuga-Wurzeln quellen dabei in Essig auf und werden anschließend getrocknet. Nach Zugabe von Salz und Wasser wird das Wasser verdampft, um eine Cimicifuga-Salz-Eindampfung zu erhalten. Hiervon werden die Cimicifuga-Wurzelbestandteile entfernt.

JP 2005/263751 A offenbart die Herstellung eines Teepulvers in Form von auflösbaren Tabletten, gewonnen durch Sprühtrocknung aus einem Extrakt von chinesischem schwarzen Tee, als Behandlung von Rauchern zur Mäßigung des Nikontin-Konsums.

EP 0 169 977 A2 befasst sich mit der Verabreichung von getrockneten Hagebutten, roten Johannisbeeren, weißen Johannisbeeren, Zitronenschalen, Grapefruitschalen und/oder Orangenschalen in Form von Tabletten, Granulat oder Würfeln zur Anwendung bei Darmverstopfung und Darmträgheit.

NL 1 033 751 C offenbart ein Kaugummi mit Teilen oder einem Extrakt von Echinacea Purpurea.

Das "Nahrungsergänzungsmittel-Verzeichnis für Deutschland NEM-2007" enthält auf den Seiten 332 bis 340, 353 und 354, 359 bis 361, 370, 379, 385, 386 und 389 eine Vielzahl von Kapseln, Kautabletten, Säften, Lutschpastillen usw., die Fruchtoder Pflanzenbestandteile enthalten.

Eine erfindungsgemäße Kautablette der eingangs erwähnten Art ist dadurch gekennzeichnet, dass im Trägermaterial getrocknete und pulverisierte Teile einer Pflanze, nämlich Blätter, Blüten, Früchte oder Wurzeln der Pflanze, ausgewählt aus einer oder mehreren Pflanzen der Pflanzengattungen Althaea (Eibisch), Baptisia (Indigo), Cetraria (Flechten), Cimicifuga (Silberkerzen), Crataegus (Weißdorn), Echinacea (Sonnenhut) Hedera (Efeu), Malphigia (Acerola), Mentha (Minze) und Rosa (Hagebutte) verteilt sind, wobei die Gesamtmasse der Tablette zwischen 300 und 2000 mg beträgt und der Anteil der getrockneten und pulverisierten Pflanzenteile zwischen 10 und 50 Gew.%, bezogen auf die Gesamtmasse der einzelnen Kautablette, beträgt.

Die erfindungsgemäße Kautablette ermöglicht somit die Zuführung von Wirkstoffen aus Pflanzen zum menschlichen Körper in einer prinzipiell von herkömmlichen Darreichungsformen unterschiedlichen Art. Pflanzenwirkstoffe werden üblicherweise als Extrakte aufbereitet und dem menschlichen Körper zugeführt. Durch die erfindungsgemäße Kautablette lässt sich die Durchführung eines Extraktionsverfahrens vermeiden und dennoch eine wirksame Zuführung von pflanzlichen Wirkstoffen über die Mund- und Rachenschleimhaut zum menschlichen Körper erreichen. Die erfindungsgemäße Kautablette enthält die Pflanzenteile, die lediglich gereinigt, getrocknet und pulverisiert werden, sodass sie bezüglich ihrer Wirkstoffe als unveränderte Pflanzenteile in der kaufähigen Tablette vorhanden sind. Die erfindungsgemäße Kautablette kann gelutscht und gekaut werden, wobei bereits im Mundraum die pflanzlichen Bestandteile freigesetzt werden. Durch den Kauvorgang und die Vermischung der pflanzlichen Bestandteile mit dem Speichel werden die pflanzlichen Wirkstoffe enzymatisch aufgeschlossen und dadurch in dosierter Form und über den Kauvorgang zunächst weitgehend gleichmäßig resorbierbar.

Im Gegensatz zu Lutschtabletten, bei denen mit zunehmender Auflösung der aktive Lutschvorgang abnimmt, erreicht die beschriebene kaufähige Darreichungsform auch eine Verteilung der Wirkstoffe, die pharmazeutische oder pflegende Bestandteile enthalten können, nach der Freisetzung durch die weiterhin mögliche Kautätigkeit. Dies führt zu einer andauernden Speichelsekretion und im Vergleich zu den herkömmlichen Darreichungsformen intensiveren und längeren Speichelsekretion und -verteilung im Mund- und Rachenraum.

Im Unterschied zu herkömmlichen Kautabletten entspricht die Masse der erfindungsgemäßen Kautablette der Massendimension einer pharmazeutisch verwendeten Tablette und liegt zwischen 300 und 2000 mg. Der Anteil der getrockneten und pulverisierten Teile einer Pflanze liegt zwischen 10 und 50 Gew.%, bezogen auf die Gesamtmasse der einzelnen Kautablette.

Die getrockneten und pulverisierten Pflanzenteile können insbesondere Blätter, Blüten, Früchte und Wurzeln sein. Darüber hinaus sind Kombinationen von Pflanzenpulvern und Extrakten einsetzbar. Dabei können auch Pflanzenpulver verschiedener Pflanzen, Extrakte verschiedener Pflanzen sowie entsprechende Mischungen von Pflanzenpulvern und Extrakten verschiedener Pflanzen berücksichtigt werden.

Die pflanzlichen Teile entstammen Pflanzen der Pflanzengattungen Althaea (Eibisch), Baptisia (Indigo), Cetraria (Flechten), Cimicifuga (Silberkerzen), Crataegus (Weißdorn), Echinacea (Sonnenhut) Hedera (Efeu), Malphigia (Acerola), Mentha (Minze) und Rosa (Hagebutte).

Die erfindungsgemäße Kautablette kann als weitere Bestandteile auch Mineralstoffe und Vitamine enthalten.

Ebenso kann die Kautablette auch mit Süßungszusätzen versehen werden. Idealerweise werden dafür Fruktose (Fruchtzucker) oder Saccharose (Kristallzucker) verwendet. Zum Einsatz können aber auch Zuckeraustauschstoffe wie Isomaltit, Maltit, Mannit, Sorbit oder Xylit sowie Süßungsstoffe wie insbesondere Acesulfam, Aspartam, Cyclamat, Saccharin und Sucralose kommen. Darüber hinaus sind Kombinationen der einzelnen Substanzen möglich.

Der Kautablette können auch Geschmacksmaskierungssubstanzen und Aromenstoffe zugesetzt werden.

Die erfindungsgemäße Kautablette kann auch befilmt werden. Die Befilmung kann dabei die oben erwähnten Süßungszusätze enthalten.

Die erfindungsgemäße Kautablette kann somit als Arzneimittel oder als Mund- bzw. Rachenpflegemittel verwendet werden, mit dem Wirkstoffe unmittelbar aus Teilen einer Pflanze im Mund- und Rachenraum extrahiert und aufgeschlossen und der Schleimhaut zugeführt werden. Die dabei freigesetzten Wirkstoffe unterscheiden sich von in pflanzlichen Arznei- und Pflegemitteln enthaltenen Extrakten aufgrund des enzymatischen Aufschlusses. Darüber hinaus steht der Wirkstoff unmittelbar nach dem Aufschluss am Resorptionsort zur Verfügung. Der Aufschlussvorgang ist dabei von dem Kauvorgang abhängig, sodass eine über eine längere Kaudauer vergleichmäßigte Resorption des Wirkstoffs bzw. der Wirkstoffe erreichbar ist.

## Patentansprüche

1. Kautablette mit einem kaufähigen, sich im Mund eines Menschen nur nach einem ununterbrochenen Kauen über mehr als eine Minute auflösenden Trägermaterial, **dadurch gekennzeichnet, dass** im Trägermaterial getrocknete und pulverisierte Teile einer Pflanze, nämlich Blätter, Blüten, Früchte oder Wurzeln der Pflanze, ausgewählt aus einer oder mehreren Pflanzen der Pflanzengattungen Althaea (Eibisch), Baptisia (Indigo), Cetraria (Flechten), Cimicifuga (Silberkerzen), Crataegus (Weißdorn), Echinacea (Sonnenhut) Hedera (Efeu), Malphigia (Acerola), Mentha (Minze) und Rosa (Hagebutte) verteilt sind, wobei die Gesamtmasse der Tablette zwischen 300 und 2000 mg beträgt und der Anteil der getrockneten und pulverisierten Pflanzenteile zwischen 10 und 50 Gew.%, bezogen auf die Gesamtmasse der einzelnen Kautablette, beträgt.

2. Kautablette nach Anspruch 1, **dadurch gekennzeichnet, dass** in Kombination mit den Pflanzenteilen ein Extrakt der Pflanze in dem Trägermaterial verteilt ist.

3. Kautablette nach Anspruch 2, **dadurch gekennzeichnet, dass** aus den Teilen und ggf. dem Extrakt gebildete pflanzliche Bestandteile der Tablette 10 bis 50 Gew.% der Gesamtmasse ausmachen.

4. Kautablette nach Anspruch 1, **dadurch gekennzeichnet, dass** die Kautablette frei von Extrakten ist.

## Claims

1. Chewable tablet with a chewable excipient material which dissolves in the mouth of a human only after continuous chewing over more than one minute, **characterized in that** dried and powdered parts of a plant, namely leaves, flowers, fruits or roots of the plant, selected from one or more plants of the plant genera Althaea (Malvaceae), Baptisia (indigo), Cetraria (lichens), Cimicifuga (cohoshes), Crataegus (hawthorn), Echinacea (coneflower), Hedera (ivy), Malphigia (acerola), Mentha (mint) and Rosa (rosehip) are distributed within the excipient material, where the total weight of the tablet amounts to between 300 and 2000 mg and the amount of the dried and powdered plant parts amounts to between 10 and 50% by weight, based on the total weight of the individual chewable tablet.

2. Chewable tablet according to Claim 1, **characterized in that** an extract of the plant is distributed within the excipient material, in combination with the plant parts.

3. Chewable tablet according to Claim 2, **characterized in that** plant constituents of the tablet, formed of the parts and, if appropriate, the extract, account for 10 to 50% by weight of the total weight.

4. Chewable tablet according to Claim 1, **characterized in that** the chewable tablet is free from extracts.

## Revendications

1. Comprimé à mâcher avec un matériau porteur capable d'être mâché et qui se dissout dans la bouche d'un être humain uniquement après avoir été mâché de façon ininterrompue pendant plus d'une minute, **caractérisé en ce que** des parties séchées et pulvérisées d'une plante, à savoir feuilles, fleurs, fruits ou racines de la plante, sélectionnée parmi une ou plusieurs plantes des espèces de plantes Althaea (guimauve), Baptisia (indigo), Cetraria (lichen), Cimicifuga (actée), Crataegus (aubépine), Echinacea (échinacée), Hedera (lierre), Malphigia (acérola), Mentha (menthe) et Rosa (églantier) sont réparties dans le matériau porteur, de sorte que la masse totale du comprimé s'élève entre 300 et 2000 mg et la part des parties de plantes séchées et pulvérisées s'élève entre 10 et 50 % en poids, par référence à la masse totale du comprimé à mâcher individuel.

2. Comprimé à mâcher selon la revendication 1, **caractérisé en ce qu'**un extrait de la plante est réparti dans le matériau porteur en combinaison avec les parties de plantes.

3. Comprimé à mâcher selon la revendication 2, **caractérisé en ce que** les composants végétaux, formés par les parties et le cas échéant l'extrait, du comprimé représentent 10 à 50 % en poids de la masse totale.

4. Comprimé à mâcher selon la revendication 1, **caractérisé en ce que** le comprimé à mâcher est dépourvu d'extraits.
